# EUROPEAN PATENT SPECIFICATION

(11) **EP 0 975 321 B1**
(45) Date of publication and mention of the grant of the patent: **02.02.2005**
(21) Application number: 98919263.8
(22) Date of filing: 14.04.1998
(51) Int. Cl.: A61K 7/48

(54) **THE USE OF THIOGLYCOLIC ACID AS DEPIGMENTING AGENT**
VERWENDUNG VON THIOGLYCOLSÄURE ALS HAUTBLEICHENDES MITTEL
UTILISATION D'ACIDE THIOGLYCOLIQUE EN TANT QU'AGENT DE DEPIGMENTATION

(30) Priority: 18.04.1997 IT MI970928
(43) Date of publication of application: 02.02.2000
(73) Proprietor: I.V. Pharma S.A.S., 10090 Romano Canavese (IT)
(72) Inventor: IZZO, Marcello, I-80030 San Paolo Belsito (IT); VERZELLA, Gianfranco, I-10090 Romano Canavese (IT)
(74) Representative: De Gregori, Antonella
(86) International application number: PCT/EP1998/002159
(87) International publication number: WO 1998/047466

(56) References cited:
- WO-A-91/02538
- WO-A-95/34280
- US-A- 4 195 095
- DATABASE WPI Week 8401 Derwent Publications Ltd., London, GB; AN 84-002869 XP002077094 "Skin bleaching composition for treating skin diseases, freckles, etc.-contains cod-liver oil, sulphur compound, hydrogen peroxide salicylic acid and acidic bromate" A & JP 58 198421 A (KOSAKA R) 18 November 1983
- DATABASE WPI Week 8210 Derwent Publications Ltd., London, GB; AN 82-18405e XP002084155 "Cosmetic for hair conditioning and preventing skin ageing-comprises sulphur-containing reducing agent, e.g. thioglycolic acid or cysteine or ascorbic acid and cop. obtd. by oxidising reducing agent" A & JP 57 016810 A (MURAI Y) 28 January 1982

## Description

The present invention relates to the use of 2-mercaptoacetic acid (thioglycolic acid) for the preparation of pharmaceutical compositions having depigmenting activity on skin spots of ferric origin (hemosiderin), which often occur during sclerotherapy or intravenous infusion administrations, as well as a cosmetic treatment of epidermal spots of ferric origin. Such spots are present on skin as more or less permanent pigmentations.

### Background of the invention

The formation of hemosiderinic (ferric) pigmentations on the treated site is a side effect given by all the sclerosing solutions, normally used in therapy. This side effect is quite general and independent from the correct use of sclerosing solutions and the technique of application of sclerotherapy. Hemosiderinic pigmentations appear in the form of dark cutaneous spots, which are felt as a serious aesthetic damage by patients, who consequently do not normally comply with this kind of therapy. These inconveniences are more frequent in small blood vessels, such as telangiectasis, which notoriously represent 80% of sclerotherapic applications. According to some Authors (M.P. Goldman, USA), the incidence of these post-sclerosis hyperpigmentation is estimated about 10-30 % of the patients undergoing sclerosing therapy independently from the type of medicament used.

Most of all, prevention of hyperpigmentation is obtained by applying a correct sclerotherapeutic technique, by selecting the appropriate type and doses of sclerosing agent. However, notwithstanding the above measures, these side effects may happen even to the most expert sclerotherapist.

Accordingly, the availability of a cosmetic or pharmaceutical agent capable of eliminating or at least reducing this aesthetic damage is still today strongly desired by the therapist and patients.

Among the several preparations having depigmenting activity, the so-called hemosiderinic solubilizing agents, for topical use, have proven to be effective and do not show undesirable side effects. These are preparations normally of cosmetic adjuvants able to "solubilize" hemosiderin.

Post-sclerotherapy pigmentations form in about 6-12 weeks from the end of therapy; they have a linear-macular morphology with a grey to dark brown colour.

Generally, colouring tends to brown in the course of some years, except in case of spontaneous resolutions.

A number of scientific proposals has been offered to explain the genesis of these pigmentations, but only in the last 20 years, experimental evidences of their exact nature have been obtained, thanks to bioptic studies of some Authors (Barner, 1977; Cuttel and Fox, 1982; Goldman, 1987) who demonstrated the almost exclusively haematic origin of the pigment associable with the protein complex called "hemosiderin".

In particular, Goldman (J. Dermatol. Surg. Oncol., 13,547.1987) studied the effect of the pigmentation genesis in patients subjected to telangectasic therapies (small blood vessels) using different agents in different conditions.

The essentially "haematic" nature of the "post-sclerotic" pigmentation was therefore widely demonstrated.

Hemosiderin, which is a base component of pigmentation, forms from the degradation of haeme in the cycle of bile genesis, and has different morphologies of complex nature, as shown by some studies (Richter, 1957 e 1965; Shoden, 1960; Wells, 1962). Genesis cycle, during sclerosing therapies, can be summarized as follows:
- sclerosing action determines erhytrodiapedesis, due to the combined effect of needle shock and alteration of blood vessel permeability: hence the subsequent degradation of haemoglobin in the tissue-site;
- consequently, there is complexation with apoferritin part of the iron released from haeme and formation of the ferritin protein complex, which represents the mobile and hydrosoluble pool of ferric storage in physiological environment;
- the subsequent degradation to hemosiderin, which is insoluble in aqueous environments, can be explained by the action of acid environment (often given by sclerosing solutions themselves, which have acidic pH) and a further degradation of ferritin and loss of the protein part apoferritin. The hemosiderin complex is water-insoluble, except at very low pH, thus allowing the organism to have available an "iron pool", which is released only in particular pH conditions.

Therefore hemosiderin would be a sort of biological advantage, because, on one hand, it would release iron only in particular conditions (pH < 4.5) as shown by O'Connel (1986, Biochem. J., 234:727-731) and, on the other hand, it would avoid the presence of free iron, which is known to be toxic.

Taking into account what above discussed, in the topical treatment of epidermal spots, one should distinguish the goal of reducing and eliminating pigmentations of melanine origin, which earlier patents relates to (South Africa patent n. 68-3773), with respect to the goal of reducing and eliminating pigmentations of haematic origin, in particular those of hemosiderinic genesis and coming from sclerotherapy and intravenous infusion treatments (blood transfusions, etc.). Reduction and elimination of skin pigmentations of haematic origin, in particular those of hemosiderinic genesis and coming from sclerotherapy and intravenous infusion treatments (blood transfusions, etc.) is the problem faced by the present invention.

As above said, contrary to ferritin, the protein complex containing trivalent iron is insoluble and accumulates in dermis permanently (particularly in papillar dermis) showing a characteristic brown spot, with remarkable unesthetic effect.

In previous years, a number of techniques, believed to be suitable to solve such essentially unaesthetic inconvenience, were proposed.

As a matter of clarity and better understanding, these techniques may be classified in instrumental techniques and in techniques using substances having physico-chemical activities.

Main instrumental techniques are the following :
1. Cryotherapy: it is a complex and expensive technique, which has the intrinsic difficulty of determining the exact deepness of frozing.
2. Dermo-Microabrasion: consists in the use of vacuum systems with mineral powders (Corundum). It gives variable results and is expensive.
3. Laser systems: two expensive kinds of apparatuses are available, but variable and not reproducible results are obtained.

Chemical substances having depigmenting activity can be divided in two classes:
1. Substances having chelating action on hemosiderin iron;
2. Substances having solubilizing action on hemosiderin.

Both classes are used in topical formulations.

In the first class ethylenediaminotetracetic acid (EDTA) is found; it shows the well-known chelating effect on metals and is used in formulations having 15% concentration (150 mg/ml).

Further, desferroxamine is a hydrosoluble sideramine capable of chelating iron ion specifically. It is used topically, but the results are not reproducible.

Among the solubilizing substances of the second class, the following are mentioned:
- alfa-hydroxyacids, in particular 2-hydroxyacetic acid (glycolic acid). This substance does not have a constant effect in its results because it is not capable of reaching the derm and, in order to obtain a medium effect, it requires the application on epidermis for very long times.

Plurihalogen acids. In particular trichloroacetic acid is mentioned, because it has a very good penetration capability in the cutis and can easily reach hemosiderin and solubilize it, due to its characteristics of very strong acid. But occurrence of side effects, such as hypopigmentations and post-phlogosis scars from chemical burning are reported. Also incidents of activation of keratocytes of the basal layer and reduction of the basal melanophores are reported. Normally, trichloroacetic acid is used in aqueous or amphiphilic solutions, with concentrations ranging from 10 to 40%.

Examining in detail the simplest and less expensive techniques, which are those using cosmetic or pharmaceutical formulations having a solubilizing action, the person having ordinary experience in the art will ascertain some drawbacks that, in one way or in another, lower the efficacy or makes their use difficult. In fact, taking into account what above stated, it can be said:
- the use of formulations containing chelating agents, such as EDTA or desferroxamine (already proposed by some authors, see Meyers in 1966 and Goldman in 1992) give variable, uncertain results, likely due to their difficult diffusion through cutis, since topical way is the most preferred;
- the selection of solubilizing substances unfortunately comprises other kinds of problems. Trichloroacetic acid, although highly effective, is dangerous because it is a strong acid and has a strong protein denaturating activity: even if it used at low concentrations or for short periods of time (but in this case efficacy is lowered), it gives rise to phlyctena, hypopigmentations due to melanocyte destruction. On the contrary, the choice of using preparations containing glycolic acid does not show these noxious effects, since it is not a strong acid, as trichloroacetic acid is, but it has a very superficial activity, as it cannot easily penetrate the epidermic-dermal junction: in order to reach the miminum efficacy, very long application times are necessary. But in this case, side effects similar to those experienced with trichloroacetic acid occur, for example hypopigmentation. Accordingly, a correct and cautious use gives only variable results.

For the reasons above discussed, the therapist is still looking for a highly effective active ingredient without exposing the patient to the risks or problems encountered in the present state of the art.

WO95/34280, JP58198421 and US4195095 disclose compositions comprising thioglycolic acid. These documents teach the use of these compositions to treat regions of high melanin content (WO95/34280) , strains, freckles, macula or melanoderma (JP58198421), acne, fatty cysts, dandruff or scleroderma (US4195095).

### Summary of the invention

It has now surprisingly been found that 2-mercaptoacetic acid (commonly named "thioglycolic acid") shows a strong and exceptional depigmenting activity and gives constant results. Advantageously, many side effects, arising from the use of the active ingredients of the art, do not appear when 2-mercaptoacetic acid is used.

Accordingly, it is an object of the present invention the use of 2-mercaptoacetic acid as active ingredient for the preparation of a pharmaceutical composition having depigmenting activity on skin spots of ferric origin.

It is another object of the present invention, a method for the cosmetic treatment of depigmentation of skin spots of ferric origin comprising the application on epidermis of a composition containing 2-mercaptoacetic acid.

The inventors do not wish to be bound to any theory, however they believe that the strong solubilizing activity at dermis level with respect to hemosiderin iron is due not only to the acidic characteristic of the active ingredient, but, above all, to the presence of -SH group, which gives the molecule a strong affinity for iron ion in the skin: then, according to the present invention, there is the advantageous result to achieve both the solubilizing effect and the complexing effect with a single active ingredient.

The present invention offers the art of sclerotherapy a number of advantages, because, other than a well established and constant efficacy, the composition is endowed with a high safety of use.
- Only very short application times are required, at most a few minutes, and it is subsequently eliminated with washing and neutralization with a buffer or simply by washing the treated site with tap water. Short application times allow to eliminate risks and dangers of harmful effects, such as erythemas, phlyctena, hypopigmentations, etc.
- It has also been found that the strong affinity for iron ion of 2-mercaptoacetic acid gives it a behaviour similar to that of apoferritin, therefore it can bind to hemosiderin even at low concentrations, thus giving higher efficacy at concentrations lower than those of glycolic acid (used at 7%) and trichloroacetic acid (used at 20-40%).

2-mercaptoacetic acid was used as chemical reagent in laboratory for the determination of hemosiderin and characterization of its structure and chemical composition (Shoden and Sturgeon in Hemosiderin; Acta Hematol., 376-392, 1960).

Other Authors showed the capability of 2-mercaptoacetic acid of eliminating iron accumulations from spleen and to have antioxidant activity (due to -SH group), for example demonstrated by its presence of metabolite of carboxymethylcysteine. (Joo et al., Biol. Met. 3(34), 1715, 1990; Hofman et al., Drug. Metab. Disps., 19(1)22-6, Jan-Feb. 1991).

Efficacy of 2-mercaptoacetic acid is due only to its characteristic of being simply an acid: in fact it has an acidity definitely lower than the one of trichloroacetic acid (a well known depigmenting agent) (see Table 1 below), in fact 2-mercaptoacetic acid is a mid-strenght acid, such as glycolic acid, and as the latter, slightly stronger than acetic acid.

Therefore, the present invention provides an active ingredient, which, although highly effective, does not give side effects. Topical use of the compositions containing 2-mercaptoacetic acid, according to the present invention, does not cause burning or erythema except rare cases, but of very low intensity; also sensitization is quite exceptional and temporary.

**Table 1 -**

| Acidic strength | | |
|---|---|---|
| Substance | Chemical formula | pKa value |
| Acetic acid | CH₃COOH | 4.76 |
| Glycolic acid | HO-CH₂COOH | 3.83 |
| Tioglycolic acid | HS-CH₂COOH | 3.68 |
| Chloroacetic acid | Cl-CH₂COOH | 2.85 |
| Trichloroacetic acid | Cl₃CCOOH | 0.70 |

Pharmaceutical and cosmetic compositions containing 2-mercaptoacetic acid as active ingredient provide the phlebologist suitable means for an effective cosmetic or therapeutic treatment without contra-indications with the scope of eliminating or at least minimizing side effects of hemosiderinic pigmentations during sclerotherapy treatments.

According to the present invention, 2-mercaptoace-tic acid shall be used in an amount higher than the minimum value of 3% by weight, thus providing a very effective therapeutical means, that in any case is to be used in a treatment in casualty department.

Compositions having definitely lower concentrations (lower than 3% by weight), are foreseen for a possible continuation of the treatment at home, in order to establish an effective maintenance treatment.

Generally, the effective amount of active ingredient is comprised from 0.5 to 30% by weight. This range of concentration is also useful to be used as an antiaging tool.

The cosmetic composition containing 2-mercaptoacetic acid to be used in the present invention shall have a pH of the aqueous base ranging from 2.5-7.0, by adjusting the aqueous solution either with basic agents (sodium, ammonium or potassium hydroxide solution) or with mixtures of suitable buffer salts (sodium or potassium phosphate salts).

The compositions to be used in the present invention are preferably in the form of hydro-alcoholic solutions or gels or ointments.

The preparation of the compositions can be carried out according to conventional techniques well-known to the skilled person.

As an example, methods for industrial manufacture are disclosed below:
1. Solutions having concentrations comprised in the 0.5-15% w/w range in 20 ml bottle and/or 2 ml ampoules.
2. Ointments and/or gels having concentrations comprised in the range 0.5-15% w/w in 20 g tube and/or 3 g sachets.

### Example 1

Formulation for the preparation of 1000 g of 12% solution, in 20 ml bottles and/or 2 ml ampoules.

| Starting materials | Amounts gram/Kg 12% solution |
|---|---|
| Thioglycolic acid 100% | 120 |
| Ethyl alcohol 96° | 200 |
| Polyethylene glycol 400 | 200 |
| Antioxidant | 1.00 |
| Perfume | 20 |
| Water : q.s. to | 1000 grams. |

In a glass vessel, equipped with stirrer, ethyl alcohol is mixed with polyethylene glycol till complete dissolution; antioxidant, then thioglycolic acid and perfume are slowly added while stirring.

Depurated water is added under stirring to the final weight in order to have a complete dissolution.

The mass is filtered, if necessary, through membrane and used for primary packaging by means of an equipment with automatic control of the distributed quantity.

Moulded glass bottles, having brown colour for light protection, are used.

Class I-glass ampoules are packaged in normal conditions, since the solutions are not for injection: ampoule filling is carried out in nitrogen atmosphere, having previoulsy purged the ampoules with nitrogen, to preserve the best stability during the time.

### Example 2

Formulation for the preparation of 100 grams 12 % of ointment and/or gel in 20 g tube and/or in 3 g sachets.

| Starting materials | Amount in grams for 12% ointment/gel | Component characteristics |
|---|---|---|
| Mercaptoacetic acid | 12.0 | Aqueous phase |
| Glycerine | 4.0 | Aqueous phase |
| Xanthan Gum | 2.0 | Aqueous phase |
| Brij 721/72 | 5 | Emulsifying |
| Cetyl/stearyl alcohol | 3.0 | Fatty phase |
| Cetiol V | 4 | Fatty phase |
| Isopropyl myristate | 3.0 | Fatty phase |
| Titanium dioxide | 2.0 | Solid phase |
| Tocopheryl acetate | 1 | Antioxidant |
| Perfume | 0.2 | Hydroalcoholic Phase |
| Preserved water | q.s. to 100 grams | Aqueous phase |

- Fatty phase:: All the fatty materials are placed in a suitable vessel and melted at the temperature of 60-70°C, keeping the mass under stirring.

While stirring, emulsifying agent (Brij), tocopheryl acetate and titanium dioxide are added to the molten mass; subsequently, temperature is lowered to about 50°C.
- Aqueous phase:: In a similar manner and into a second suitable vessel, glycerine and the active ingredient (mercaptoacetic acid) are dissolved in water keeping the mixture under continuous stirring, until complete dissolution. Perfume is added, then the mass is warmed to about 50°C.

The aqueous phase is subsequently percolated into the fatty phase, kept under stirring with the usual technique for the preparation of oil/water emulsions, cooled down to a temperature such that to maintain the fluidity necessary for the subsequent filling phase in the primary packaging, which can consist of tubes (plastic or metal) or sachets of polycoupled material.

## Claims

1. The use of 2-mercaptoacetic acid as active ingredient for the preparation of a medicament having depigmenting activity on skin spots of ferric origin.

2. The use according to claim 1, wherein the amount of active ingredient is comprised between 0.5 and 30% by weight.

3. The use according to claim 1, wherein the amount of active ingredient is at least 3% by weight.

4. The use according to claim 1, wherein the amount of active ingredient is less than 3% by weight.

5. A method for the cosmetic treatment of epidermal spots of ferric origin comprising the application on epidermis of a composition containing 2-mercaptoacetic acid as active ingredient in admixture with conventional vehicles and excipients.

6. A method according to claim 5, wherein the active ingredient is contained in an amount comprised between 0.5 and 30% by weight.

7. A method according to claim 5, wherein the active ingredient is contained in an amount of at least 3% by weight.

8. A method according to claim 5, wherein the active ingredient is contained in an amount of less than 3% by weight.

## Patentansprüche

1. Die Verwendung von 2-Mercaptoessigsäure als Wirkstoff für die Herstellung eines Medikaments mit depigmentierender Aktivität auf von Eisen herrührenden Hautpunkten.

2. Die Verwendung gemäß Anspruch 1, worin die Menge des Wirkstoffes zwischen 0,5 und 30 Gew.-% umfasst.

3. Die Verwendung gemäß Anspruch 1, worin die Menge des Wirkstoffes wenigstens 3 Gew.-% ist.

4. Die Verwendung gemäß Anspruch 1, worin die Menge des Wirkstoffes weniger als 3 Gew.-% ist.

5. Verfahren für die kosmetische Behandlung von epidermalen von Eisen herrührenden Punkten, welches die Applikation einer Zusammensetzung, die 2-Mercaptoessigsäure als Wirkstoff in Abmischung mit konventionellen Carriern und Arzneiträgern enthält, auf der Epidermis umfasst.

6. Verfahren gemäß Anspruch 5, wobei der Wirkstoff in einer Menge enthalten ist, die zwischen 0,5 und 30 Gew.-% umfasst.

7. Verfahren gemäß Anspruch 5, worin der Wirkstoff in einer Menge von wenigstens 3 Gew.-% enthalten ist.

8. Verfahren gemäß Anspruch 5, worin der Wirkstoff in einer Menge von weniger als 3 Gew.-% enthalten ist.

## Revendications

1. Utilisation de l'acide 2-mercaptoacétique en tant que principe actif pour la préparation d'un médicament ayant une activité de dépigmentation sur les tâches cutanées d'origine ferrique.

2. Utilisation selon la revendication 1, dans laquelle la quantité de principe actif est comprise entre 0,5 et 30 % en poids.

3. Utilisation selon la revendication 1, dans laquelle la quantité de principe actif est d'au moins 3 % en poids.

4. Utilisation selon la revendication 1, dans laquelle la quantité de principe actif est inférieure à 3 % en poids.

5. Procédé de traitement cosmétique des tâches épidermiques d'origine ferrique, comprenant l'application sur l'épiderme d'une composition contenant de l'acide 2-mercaptoacétique en tant que principe actif mélangé à des véhicules et à des excipients conventionnels.

6. Procédé selon la revendication 5, dans lequel le principe actif est contenu en une quantité comprise entre 0,5 et 30 % en poids.

7. Procédé selon la revendication 5, dans lequel le principe actif est contenu en une quantité d'au moins 3 % en poids.

8. Procédé selon la revendication 5, dans lequel le principe actif est contenu en une quantité inférieure à 3 % en poids.
